# EUROPEAN PATENT APPLICATION

(11) **EP 4 494 645 A1**
(43) Date of publication of application: **22.01.2025**
(21) Application number: 23770891.2
(22) Date of filing: 16.03.2023
(51) Int. Cl.: A61K 35/768, A61P 35/00, A61P 35/04, C07K 14/705, C12N 5/09, C12N 7/00, C12N 15/45

(54) **NOVEL USE OF ONCOLYTIC GENE-MODIFIED MEASLES VIRUS**

(30) Priority: 16.03.2022 JP 2022041183
(71) Applicant: The University of Tokyo, Bunkyo-ku, Tokyo 113-8654 (JP)
(72) Inventor: KAI Chieko, Tokyo 113-8654 (JP); YONEDA Misako, Tokyo 113-8654 (JP); FUJIYUKI Tomoko, Tokyo 113-8654 (JP); MORITOH Kanako, Tokyo 113-8654 (JP)
(74) Representative: De Vries & Metman
(86) International application number: PCT/JP2023/010385
(87) International publication number: WO 2023/176938

(57) **Abstract**

An object of the present invention is to develop gene-modified measles viruses (rMV-SLAMblind and rMV-V(-)-SLAMblind) that do not recognize SLAM as a tool for a medical treatment for tumors. The present invention provides a pharmaceutical composition for inducing cell-mediated immunity against a tumor cell to subject the tumor cell to medical treatment, the pharmaceutical composition containing an oncolytic gene-modified measles virus, and also provides a method of inducing cell-mediated immunity against a tumor cell remaining in a living body that could not be eliminated even by direct introduction of an oncolytic gene-modified measles virus, or against a tumor cell that has metastasized or recurred, the method including directly introducing an oncolytic gene-modified measles virus into a tumor cell as a medical treatment target to cause cell death.

## Description

### Technical Field

The present invention relates to a novel usage of an oncolytic gene-modified measles virus, which increases the effectiveness of the oncolytic gene-modified measles virus. Specifically, the present invention relates to a novel usage of an oncolytic gene-modified measles virus, a pharmaceutical composition using the novel usage, and a novel medical treatment method for a tumor.

Priority is claimed on Japanese Patent Application No. 2022-041183, filed March 16, 2022, the content of which is incorporated herein by reference.

### Background Art

Oncolytic viral therapy is considered to be very promising as a new strategy for the medical treatment of tumors that can be combined with traditional medical treatment methods. Currently, a wide range of viruses derived from a large number of virus species are being evaluated as oncolytic drugs, and clinical trials have been carried out for some viruses.

Measles virus (MV) is a pathogenic virus belonging to the genus Morbillivirus in the family Paramyxoviridae that infects humans as a natural host, proliferates entirely in the cytoplasm (that is, the viral sequence is not integrated into the host's chromosomal DNA), and causes immunosuppression and respiratory symptoms. Since it has been found that the measles virus infects tumor cells and induces the regression of the tumor

(Non-Patent Document 1), the measles virus has attracted attention as a tool for viral therapy of tumors.

Utilizing this feature, the research group of the inventors of the present invention produced, based on a wild-type measles virus HL strain, a recombinant measles virus (rMV-SLAMblind) that does not recognize a signaling lymphocyte activation molecule (SLAM), which is a receptor that is required for the wild-type measles virus to exhibit pathogenicity (Patent Document 1 and Non-Patent Document 1). In this document, the inventors show that, in a case where rMV-SLAMblind is allowed to infect breast cancer cells in vitro or in vivo, the breast cancer cells can be killed, and that the anti-tumor activity thereof is higher than that of the measles virus vaccine strain in the related art. In addition, it was shown that rMV-SLAMblind is completely attenuated and was also confirmed to have high safety without causing typical clinical symptoms of measles even in a case of being subcutaneously inoculated to a monkey (Patent Document 1 and Non-Patent Document 1).

Further, the research group of the inventors of the present invention found that the created rMV-V(-)-SLAMblind as a gene-modified measles virus does not recognize SLAM and found that rMV-V(-)-SLAMblind can also be used for the medical treatment of breast cancer and metastasized cancer (Patent Document 2). Furthermore, it was found that all the gene-modified measles viruses that do not recognize SLAM (rMV-SLAMblind and rMV-V(-)-SLAMblind) have an effect on the regression of the tumor not only by intratumoral administration but also by intravenous administration (Patent Document 2).

### Citation List

### Patent Documents

Patent Document 1: Japanese Unexamined Patent Application, First Publication No. 2013-216609
Patent Document 2: PCT International Publication No. WO2016/047645

### Non-Patent Document

Non-Patent Document 1: Sugiyama et al., Gene Therapy, 2013, vol.20, p.338-347.

### Summary of Invention

### Technical Problem

Oncolytic gene-modified measles viruses (rMV-SLAMblind and rMV-V(-)-SLAMblind) are characterized by their ability to infect and lyse tumor cells, thereby inducing tumor tissue regression. However, for tumor cells that cannot be directly infected with the virus, for example, deep-seated tumors or metastatic tumors that occur after medical treatment, it is necessary to develop a method other than a method of directly infecting the tumor cells. Therefore, an object of the present invention is to develop gene-modified measles viruses (rMV-SLAMblind and rMV-V(-)-SLAMblind) that do not recognize SLAM as a tool for a medical treatment for tumors.

### Solution to Problem

The inventors of the present invention discovered that induction of cell death in tumor cells into which an oncolytic gene-modified measles virus has been introduced can result in the induction of strong cellular immunity against tumor cells systemically, thereby demonstrating that the above-mentioned problem can be solved.

More specifically, the present application provides the following inventions in order to solve the above-described problems.
[1] A pharmaceutical composition for inducing a cell-mediated immunity against a tumor cell to subject the tumor cell to a medical treatment, the pharmaceutical composition containing:
   an oncolytic gene-modified measles virus.
[2] The pharmaceutical composition according to [1], in which the tumor cell as a medical treatment target is a tumor cell expressing PVRL4/Nectin4.
[3] The pharmaceutical composition according to [1] or [2], in which a tumor as a medical treatment target is breast cancer, lung cancer, colon cancer, or pancreatic cancer.
[4] The pharmaceutical composition according to any one of [1] to [3], in which the tumor cell as a medical treatment target is a tumor cell that remains without being eliminated even after direct introduction of the oncolytic gene-modified measles virus.
[5] The pharmaceutical composition according to [4], in which the remaining tumor cell is selected from the group consisting of a deep-seated tumor cell, a recurrent tumor cell, and a metastasized tumor cell.
[6] The pharmaceutical composition according to any one of [1] to [5], in which the oncolytic gene-modified measles virus is rMV-SLAM-blind or rMV-V(-)-SLAM-blind.
[7] The pharmaceutical composition according to any one of [1] to [6], in which the oncolytic gene-modified measles virus is used by intravenous administration, direct administration to a tumor mass, or intraperitoneal administration.
[8] The pharmaceutical composition according to any one of [1] to [7], in which the oncolytic gene-modified measles virus also induces the cell-mediated immunity in addition to an oncolytic action.
[9] A method of inducing cell-mediated immunity against a tumor cell remaining in a living body that could not be eliminated even by direct introduction of an oncolytic gene-modified measles virus, the method including:
   directly introducing an oncolytic gene-modified measles virus into the tumor cell as a medical treatment target to cause cell death.
[10] The method according to [9], in which the remaining tumor cell is selected from the group consisting of a deep-seated tumor cell, a recurrent tumor cell, and a metastasized tumor cell.
[11] The method according to [9] or [10], in which the oncolytic gene-modified measles virus is rMV-SLAM-blind or rMV-V(-)-SLAMblind.

### Advantageous Effects of Invention

The oncolytic gene-modified measles viruses (rMV-SLAMblind and rMV-V(-)-SLAMblind) used in the present invention, not only directly induce remarkable cell death in tumor cells by being introduced into the tumor (direct anti-tumor effect) but also induce indirect cell-mediated immunity against tumor cells that have not been directly infected with the virus (metastasized tumor cells, deep-seated tumor cells that spread to a site where the virus cannot be directly infected, and the like), whereby they can have an effect of suppressing tumor growth (secondary anti-tumor effect).

### Brief Description of Drawings

FIG. 1 is a view showing an experimental protocol for examining a secondary anti-tumor effect of an oncolytic gene-modified measles virus.
FIG. 2 is a view summarizing an increase in a tumor volume in a case where the oncolytic virus was administered. In FIG. 2, the left figure shows the tumor growth on a side where the virus was administered (left subcutaneous region) compared with a group administered with MOCK (negative control), and the right figure shows the tumor growth on a side where the virus was not administered (right subcutaneous region) compared with the group administered with MOCK (negative control).
FIG. 3 is a view showing an experimental protocol for examining an action mechanism of the secondary anti-tumor effect of the oncolytic gene-modified measles virus.
FIG. 4 is a view summarizing an increase in a tumor volume in a case where the oncolytic virus was administered. In FIG. 2, the left figure shows the tumor growth on a side where the virus was administered (left subcutaneous region) compared with a group administered with MOCK (negative control), and the right figure shows the tumor growth on a side where the virus was not administered (right subcutaneous region) compared with the group administered with MOCK (negative control).
FIG. 5 is a view showing results obtained by detecting the genome of the oncolytic virus in a tumor tissue.
FIG. 6 is a view showing the number of tumor-infiltrating immune cells in a tumor tissue on a side where the virus was administered and a tumor tissue on a side where the virus was not administered.
FIG. 7 is a view showing the number of activated T cells in a tumor tissue on a side where the virus was administered and a tumor tissue on a side where the virus was not administered.
FIG. 8 is a view showing results obtained by measuring the number of cells that highly express an activation marker CD44 on NK cells, NK T cells, CD4 T cells, and CD8 T cells, which were present in a lymph node in the vicinity of the tumor (draining LN).
FIG. 9 is a view showing the number of immune cells in the draining lymph node in the vicinity of the tumor tissue on the side where the virus was administered and the draining lymph node in the vicinity of the tumor tissue on the side where the virus was not administered.
FIG. 10 is a view showing the number of various immune cells in the spleen tissue of a mouse to which the oncolytic virus was administered.
FIG. 11 is a view showing the number of various immune cells in the spleen tissue of a mouse to which the oncolytic virus was administered.
FIG. 12 is a view showing an experimental protocol for examining a secondary anti-tumor effect of an oncolytic gene-modified measles virus.
FIG. 13 is a view showing that an inoculation treatment with the oncolytic gene-modified measles virus induces a tumor antigen-specific CD8+ T cell response.
FIG. 14 is a view showing results obtained by investigating the expression of Nectin-4 on a cell surface of a scirrhous gastric cancer cell line and a non-scirrhous gastric cancer cell line.
FIG. 15 is a view showing results obtained by investigating the infection sensitivity of the scirrhous gastric cancer cell line and the non-scirrhous gastric cancer cell line with respect to the oncolytic virus rMV-SLAMblind.
FIG. 16 is a view showing results obtained by investigating the cell survival rate of the scirrhous gastric cancer cell line and the non-scirrhous gastric cancer cell line after being infected with the oncolytic virus rMV-SLAMblind.
FIG. 17 is a view showing results obtained by investigating the anti-tumor activity of the oncolytic virus rMV-SLAMblind in a subcutaneous xenotransplantation model of HSC60 cells and HSC43 cells as an example.
FIG. 18 is a view showing results obtained by investigating the oncolytic activity of the oncolytic virus in a model in which HSC-60 cells expressing luciferase (HSC60-Luc) have been peritoneally metastasized.
FIG. 19 is a view summarizing temporal changes in tumor volume in a case where the oncolytic virus and an antibody against each immune response cell were administered to an immunocompetent mouse in which tumor cells have been transplanted in Example 8.
FIG. 20 is a view summarizing temporal changes in survival rate in a case where the oncolytic virus and an antibody against each immune response cell were administered to an immunocompetent mouse in which tumor cells have been transplanted in Example 8.

### Description of Embodiments

Oncolytic viruses are very promising as novel therapeutic agents for tumors, and can be used in combination with a medical treatment method used in the related art.
The inventors of the present invention discovered that the oncolytic gene-modified measles viruses (rMV-SLAMblind and rMV-V(-)-SLAMblind), which are used in the present invention, can have an effect of suppressing tumor growth by not only directly inducing remarkable cell death in the tumor cells by being directly introduced into the tumor cells (direct anti-tumor effect) but also inducing indirect but a strong cell-mediated immunity against tumor cells that have not been directly infected with the virus (metastasized tumor cells, deep-seated tumor cells that spread to a site where the virus cannot be directly infected, and the like) (secondary anti-tumor effect), thereby completing the present invention.

### <Pharmaceutical composition>

According to one aspect, the present invention provides a pharmaceutical composition for inducing cell-mediated immunity against a tumor cell to subject the tumor cell to a medical treatment, where the pharmaceutical composition contains an oncolytic gene-modified measles virus. The present invention provides a method of carrying out a medical treatment for tumor cells, with a method other than the method of directly infecting the tumor cells which cannot be directly infected with the oncolytic gene-modified measles virus, for example, deep-seated tumor cells, metastatic tumor cells generated after a medical treatment, or recurrent cancer cells.

The term "oncolytic gene-modified measles virus" used in the present invention refers to a measles virus obtained by subjecting a measles virus having oncolytic activity to such a gene modification that enhances safety or such a gene modification that enhances the infection efficiency to tumor cells. For example, a wild-type measles virus (MV HL strain) found to efficiently infect various tumor cells expressing PVRL4/Nectin4, proliferate in the cells, and cause efficient cell death can be used as the oncolytic gene-modified measles virus used in the present invention.

The gene modification used for the creation of the oncolytic virus according to the present invention is preferably a gene modification by which tumor cells are allowed to be infected but normal cells are not allowed to be infected, and it includes, for example, a gene modification by which the ability to bind to SLAM is made deficient so that the infectivity to SLAM-positive cells is lost. By deleting the ability to bind to SLAM, the oncolytic virus of the present invention does not infect immune system cells that are normal target cells of the measles virus and are SLAM-positive, and in infection experiments in monkeys, SLAM-deficient viruses do not cause measles pathology (Patent Document 1 and Non-Patent Document 1). In addition, the loss of infectivity to immune cells makes it possible to improve the efficacy and safety during medical treatment.

Examples of the oncolytic gene-modified measles virus used in the present invention are not particularly limited and as long as they have the features of the oncolytic gene-modified measles virus described. Examples of the oncolytic gene-modified measles virus include rMV-SLAM-blind or rMV-V(-)-SLAM-blind, produced by the inventors of the present invention previously and shown to have a direct anti-tumor effect.

"rMV-SLAMblind" which is an example of the oncolytic gene-modified measles virus used in the present invention is the oncolytic gene-modified measles virus described in Patent Document 1 and Patent Document 2. This virus strain is a gene-modified measles virus strain created as a recombinant MV that cannot selectively use SLAM, using a reverse genetics method. This gene-modified measles virus strain has lost its infectivity to SLAM-positive cells, and originally, it does not infect CD46-positive cells and induce a cytotoxic effect on the cells. However, since the gene-modified measles virus strain uses PVRL4/Nectin4 as a receptor for infecting the cells, it specifically infects PVRL4-positive tumor cells and induces their cell death. Therefore, it has oncolytic activity specific to SLAM-negative tumor cells (for example, breast cancer cells).

"rMV-V(-)-SLAMblind" which is another example of the oncolytic gene-modified measles virus used in the present invention is the oncolytic gene-modified measles virus described in Patent Document 2. This virus strain is a gene-modified measles virus strain that basically has the same oncolytic properties as the oncolytic properties of "rMV-SLAMblind" although this virus has an additional modification in the P gene of "rMV-SLAMblind". The gene-modified measles virus strain has the same features as those of rMV-SLAMblind strain (infection specificity and oncolytic activity), and, at the same time, has a feature of a gene modification such that the V gene involved in pathogenicity is not expressed, thereby being attenuated.

Specifically, rMV-SLAMblind is obtained by carrying out a substitution at the 533rd amino acid residue of the amino acid sequence of the H protein of the measles virus strain, by substituting arginine in the wild-type strain or the like with alanine. On the other hand, rMV-V(-)-SLAMblind is obtained by substituting arginine of the 533rd amino acid residue of the amino acid sequence of the H protein with alanine and further carrying out substitutions at each of the 687th and 690th bases in the P gene by substituting C with U and substituting U with C.

rMV-SLAMblind can be obtained by, for example, using a plasmid pMV-HL(7+) encoding the full-length antigenomic cDNA of the measles virus HL wild-type strain to prepare a vector (pMV-SLAMblind) in which arginine of the 533rd amino acid residue of the H protein is substituted with alanine, and preparing rMV-SLAMblind according to the reverse genetics method using the prepared vector. The full-length antigenomic cDNA of the measles virus HL wild-type strain used here, in which arginine of the 533rd amino acid residue is substituted with alanine, contains a base sequence set forth in SEQ ID NO: 1. Among the proteins encoded by this base sequence, the N protein consists of an amino acid sequence set forth in SEQ ID NO: 2, the P protein consists of an amino acid sequence set forth in SEQ ID NO: 3, the M protein consists of an amino acid sequence set forth in SEQ ID NO: 4, the F protein consists of an amino acid sequence set forth in SEQ ID NO: 5, the H protein consists of an amino acid sequence set forth in SEQ ID NO: 6, and the L protein consists of an amino acid sequence set forth in SEQ ID NO: 7. These proteins constitute the virus.

On the other hand, rMV-V(-)-SLAMblind can be obtained by, for example, using a plasmid pMV-HL(7+) encoding the full-length antigenomic cDNA of the measles virus HL wild-type strain to prepare a vector (pMV-V(-)SLAMblind) in which mutations are introduced by carrying out substitutions at two sites (by substituting U with C at the 687th site and substituting C with U at the 690th site) of the P gene of the vector (pMV-SLAMblind) in which arginine of the 533rd amino acid residue of the H protein is substituted with alanine, and preparing rMV-V(-)-SLAMblind according to the reverse genetics method using the prepared vector. It should be noted that the amino acid sequence of the protein encoded by pMV-V(-)SLAMblind is exactly the same as that of rMV-SLAMblind.

The original virus strain for producing MV-SLAMblind or rMV-V(-)-SLAMblind may be a strain other than the wild-type MV-HL strain. As a result, rMV-SLAMblind or rMV-V(-)-SLAMblind, which is used as the oncolytic gene-modified measles virus in the present invention, is not limited to those that use the pMV-SLAMblind vector or the pMV-V(-)SLAMblind described above.

The tumor cell as a medical treatment target of the pharmaceutical composition according to the present invention is characterized by being a tumor cell expressing PVRL4/Nectin4 and is preferably a tumor cell that highly expresses VRL4/Nectin4. Examples of the tumor cell that highly expresses PVRL4/Nectin4 include cells of breast cancer, lung cancer, colorectal cancer such as colon cancer, and epithelial cancer such as ovarian cancer. However, the tumor cell is not limited to these as long as it highly expresses PVRL4/Nectin4.

In the present invention, particularly, the oncolytic gene-modified measles virus used in the present invention is preferably one that efficiently infects various tumor cells expressing PVRL4/Nectin4, and thus tumor cells expressing PVRL4/Nectin4 (cells of breast cancer, lung cancer, colorectal cancer such as colon cancer, pancreatic cancer, or the like) are preferable as a medical treatment target of the pharmaceutical composition according to the present invention.

In a case of being directly introduced into tumor cells, the oncolytic gene-modified measles virus used in the present invention has an effect of significantly suppressing an increase in the tumor mass formed by the tumor cells. Such a response in which the oncolytic gene-modified measles virus used in the present invention is directly introduced into the tumor cells to cause cell death is referred to as a "direct anti-tumor effect".

In the related art, it has been suggested that, in a case where a tumor is subjected to a medical treatment using an oncolytic gene-modified measles virus, there is a possibility that tumor cells (deep-seated tumor cells, recurrent tumor cells, metastasized tumor cells, and the like) are not completely eliminated by the direct anti-tumor effect and remain in a living body. There has been a demand for the development of a method of exhibiting effectiveness in the medical treatment of such remaining tumor cells or tumors.

The present invention has shown that, by directly introducing the oncolytic gene-modified measles virus into a target tumor or target tumor cells, it is possible to not only induce cell death of tumor cells (direct anti-tumor effect) but also, as a result of the cell death thereof, induce cell-mediated immunity against tumor cells in the living body by the action of the cell-mediated immune system in the living body. The action of cell-mediated immunity makes it possible to eliminate tumor cells that cannot undergo cell death by the direct anti-tumor effect but remain in the living body, among the tumor cells as a medical treatment target. In the present invention, an action of eliminating the tumor cells remaining in the living body by cell-mediated immunity is referred to as a "secondary anti-tumor effect".

Based on this action, the present invention provides a pharmaceutical composition for inducing cell-mediated immunity against a tumor to subject the tumor to a medical treatment. That is, the medical treatment target of the pharmaceutical composition according to the present invention includes tumor cells (including deep-seated tumor cells, recurrent tumor cells, metastasized tumor cells, and the like) that remain even after the medical treatment with an anti-tumor agent having a direct anti-tumor effect in the primary lesion of the tumor; however, the medical treatment target is not limited thereto. Although many treatments for tumors have been developed and various antitumor agents are available, there are still many intractable tumors, and metastatic tumors in particular are highly malignant and there is a high demand for treatments.

In a case where the oncolytic gene-modified measles virus used in the present invention is directly introduced into a target tumor or target tumor cells, it is possible to not only induce cell death of the tumor cells (direct anti-tumor effect) but also induce cell-mediated immunity against the tumor cells in the living body. In order to achieve this object, the oncolytic gene-modified measles virus used in the present invention is administered to a subject by a method that is capable of causing the direct anti-tumor effect. The oncolytic gene-modified measles virus used in the present invention can be administered by intravenous administration, direct administration to a tumor mass, intraperitoneal administration, or the like.

Regarding the pharmaceutical composition according to the present invention, only the oncolytic gene-modified measles virus (for example, rMV-SLAMblind or rMV-V(-)-SLAMblind), which is an active ingredient, may be administered; however, it can also be used in combination with one or more other active ingredients.

The formulation form of the pharmaceutical composition may be any formulation form used in the related technical field as long as it is suitable as a form for administering the oncolytic virus. For example, the pharmaceutical composition can be used as a liquid pharmaceutical preparation or the like.

In addition, in a case of administering the pharmaceutical composition according to the present invention, it may be provided as various unit doses. The unit dose refers to a content of a predetermined amount of the recombinant measles virus according to the present invention, and the unit dose may be administered at one time as a single administration or may be provided as continuous administration over a set period. A specific unit dose can be set to a unit dose that has already been examined for a direct anti-tumor effect (Patent Document 2).

### <Method of inducing cell-mediated immunity against tumor cells>

In another aspect, the present invention also provides a method of inducing cell-mediated immunity against a tumor cell that remains in a living body (secondary anti-tumor effect), which could not be eliminated even by direct introduction of an oncolytic gene-modified measles virus, where the method includes directly introducing an oncolytic gene-modified measles virus into a tumor cell as a medical treatment target to cause cell death of the tumor cell (direct anti-tumor effect).

In general, cell-mediated immunity (secondary anti-tumor effect) against tumor cells is considered to be caused according to the following mechanism: · an antigen derived from a tumor cell is incorporated by an antigen presenting cell (APC; a macrophage or a dendritic cell) and is degraded into a small peptide,
· the degraded antigen fragment is bound to the MHC class II molecule in the endosome to be transferred to the surface of the antigen presenting cell and then is bound to the T cell receptor (TCR) of the helper T cell, whereby an antigenic determinant is presented,
· as a result, the helper T cell is activated, and then the cytotoxic T cell is activated by IL-2 or interferon y (IFN-γ), which is produced by the Th1 cell,
· the cytotoxic T cell recognizes an antigen peptide presented by the tumor cell, which has formed a complex with the MHC class I, and the cytotoxic T cell captures it as a foreign substance, thereby being activated,
· as a result, the cytotoxic T cell secretes a protein called perforin to the recognized tumor cell and induces apoptosis by making a hole in a target tumor cell and then injecting an enzyme called granzyme into the target tumor cell, or the cytotoxic T cell destroys the target tumor cell through a Fas-Fas ligand system, and
· further, the macrophage is also activated by being stimulated by the helper T cell and then has a stronger phagocytic action to engulf a tumor cell that has been attacked by the killer T cell.

Even in the method according to the present invention, it is considered to be necessary, although not being directly confirmed, that an antigen derived from a tumor cell is presented to an antigen presenting cell (APC; a macrophage or a dendritic cell) (that is, to cause a direct anti-tumor effect). In the method according to the present invention, it is conceived that in accordance with the direct anti-tumor effect, the oncolytic gene-modified measles virus used in the present invention is directly introduced into tumor cells as a medical treatment target to cause cell death, an antigen derived from the tumor cells in which cell death has occurred is incorporated by antigen presenting cells, which is accompanied by the activation of effector cells such as macrophages, cytotoxic T cells (CTL and killer T cells), and natural killer cells (NK cells), and as a result, it is possible to induce cell-mediated immunity against tumor cells into which the oncolytic gene-modified measles virus used in the present invention is not directly introduced. In particular, activated killer T cells play an important role in the secondary anti-tumor effect due to the oncolytic gene-modified measles virus used in the present invention.

In cell-mediated immunity induced in the living body, two kinds of memory T cells, that is, central memory (CM) T cells and effector memory (EM) T cells are allowed to survive for a long period of time, which makes it possible to trigger a rapid immune response even in a case where the same antigen (for example, a pathogen) reappears.

It has been revealed that the memory of cell-mediated immunity is established in the living body in a case where the direct anti-tumor effect is caused by the method according to the present invention, even in a case where tumor cells which will be recurrent in the future start to proliferate again. Therefore, it is possible to induce again cell-mediated immunity against the tumor cells in the living body without administering the oncolytic gene-modified measles virus used in the present invention. As a feature of the measles virus, it is known that the measles virus induces a strong cell-mediated immunity and cell-mediated immunity lasts for a long time to the extent that it can be said to be lifelong immunity. Therefore, this immunological effect makes it possible to expect a long-term and persistent effect of suppressing even metastatic cancer and recurrent cancer.

### <Medical treatment method for tumor through cell-mediated immunity against tumor cell>

In another aspect of using the method of inducing cell-mediated immunity against tumor cells, the present invention also provides a medical treatment method for a tumor through cell-mediated immunity against a tumor cell remaining in a living body (secondary anti-tumor effect) that could not be eliminated even by direct introduction of an oncolytic gene-modified measles virus, where the method includes directly introducing an oncolytic gene-modified measles virus into a tumor cell as a medical treatment target to cause cell death of the tumor cell (direct anti-tumor effect).

The medical treatment method according to the present invention makes it possible to medically treat tumor cells (deep-seated tumor cells, recurrent tumor cells, metastasized tumor cells, and the like) which are not completely eliminated even by the direct anti-tumor effect and remain in the living body, by the secondary anti-tumor effect against the tumor cells that remain in the living body and could not be eliminated by the direct anti-tumor effect.

This method makes it possible to medically treat tumor cells (deep-seated tumor cells, recurrent tumor cells, metastasized tumor cells, and the like) which are not completely eliminated even by the direct anti-tumor effect and remain in the living body, by the secondary anti-tumor effect against tumor cells that remain in the living body and could not be eliminated by the direct anti-tumor effect, where the tumor cells could not be eliminated by cell-mediated immunity that occurs in the living body as a result of the direct anti-tumor effect. Since such a therapeutic effect can be obtained, the medical treatment method according to the present invention is suitable as a medical treatment method for tumors that have progressed to stage 2 to stage 4 or recurrent tumors.

The medical treatment method according to the present invention may be applied to tumor patients after having receiving other cancer therapy treatments such as drug therapy, radiation therapy, and surgical therapy, or may be applied to tumor patients before receiving other cancer therapy treatments. For example, the medical treatment method according to the present invention is suitable as a first-choice therapeutic method for cancer therapy.

### [Examples]

Hereinafter, the present invention will be specifically shown with reference to Examples. The following Examples are not intended to limit the present invention to any particular method.

### [Example 1] Examination of anti-tumor effect of oncolytic gene-modified measles virus

The present example is an example in which, in order to examine the anti-tumor effect of the oncolytic gene-modified measles virus used in the present invention, the growth of tumor cells on a side where the virus was administered and the growth of tumor cells on a side where the virus was not administered were investigated in a case where the oncolytic gene-modified measles virus used in the present invention was administered using an immunocompetent mouse transplanted with tumor cells derived from a mouse lymphoma.

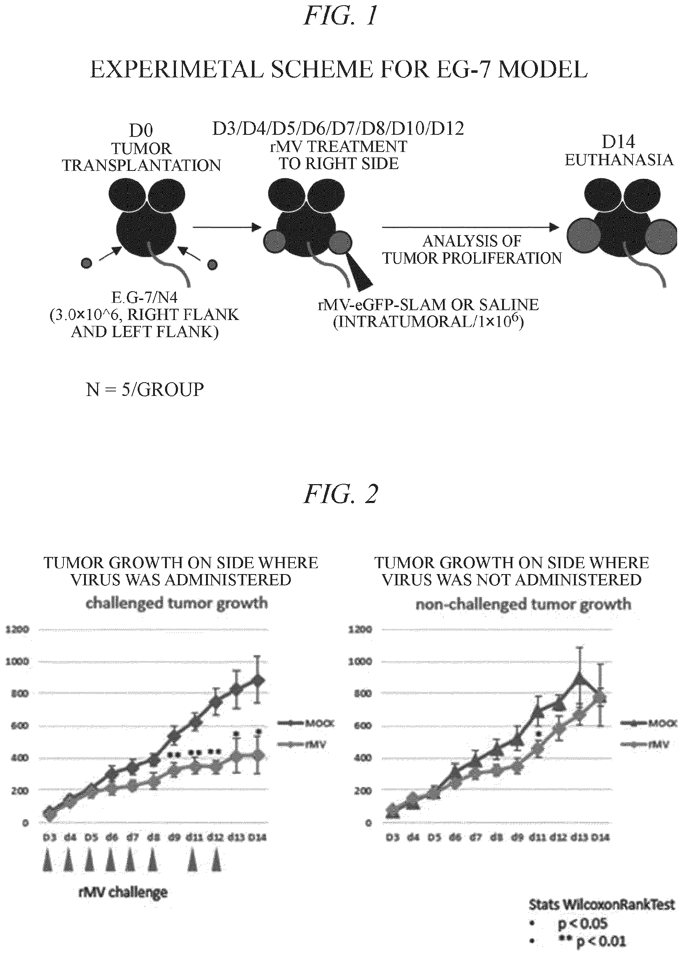

The protocol of the experiment is shown in FIG. 1. Specifically, 3 × 10⁶ E.G-7 cells (a T cell lymphoma cell line, ova-expressing tumor, obtained from ATCC) or B16 cells (a melanoma cell line, obtained from ATCC) were subcutaneously transplanted into both sides of the flank region of C57BL/6J mice (n = 5) (the day of transplantation was denoted as D0). Thereafter, while raising the mice in a clean environment, 1 × 10⁶ TCID₅₀ of rMV-SLAMblind (rMV-EGFP-SLAMblind) retaining an EGFP gene, or saline as a negative control, was administered only to the inside of the right transplantation site on each day at 3 days after transplantation (D3), 4 days after transplantation (D4), 5 days after transplantation (D5), 6 days after transplantation (D6), 7 days after transplantation (D7), 8 days after transplantation (D8), 10 days after transplantation (D10), and 12 days after transplantation (D12) after transplantation, but nothing was administered to the right transplantation site. rMV-EGFP-SLAMblind was prepared according to the method already reported (Patent Document 1). Regarding the size of the tumor, the major and minor axes were measured with a caliper up to D14, and the volume thereof was determined by calculation.

The increase in the tumor volume is summarized in FIG. 2. In FIG. 2, the left figure shows the tumor growth on a side where the virus was administered (left subcutaneous region) compared with a group administered with MOCK (negative control), and the right figure shows the tumor growth on a side where the virus was not administered (right subcutaneous region) compared with the group administered with MOCK (negative control).

It was shown that the tumor volume on the side where the virus was administered was significantly suppressed by the inherent action of the oncolytic virus (the direct anti-tumor effect, left in FIG. 2). On the other hand, the tumor volume on the side where the virus was not administered, the increase in the tumor volume was suppressed even though the oncolytic virus was not administered into the tumor (right in FIG. 2). It was considered that the oncolytic virus (rMV-SLAMblind) used in this example will not be circulated in the blood in the living body. Therefore, it was considered that the increase in the tumor volume on the side where the virus was not administered was suppressed through a mechanism other than the direct administration of the oncolytic virus.

### [Example 2] Examination of mechanism of anti-tumor effect of oncolytic gene-modified measles virus in non-virus-administered tumor tissue (1)

The present example was carried out to examine the action mechanism of the anti-tumor effect of the oncolytic gene-modified measles virus used in the present invention, at the non-virus-administered tumor tissue, where the anti-tumor effect was revealed in Example 1.

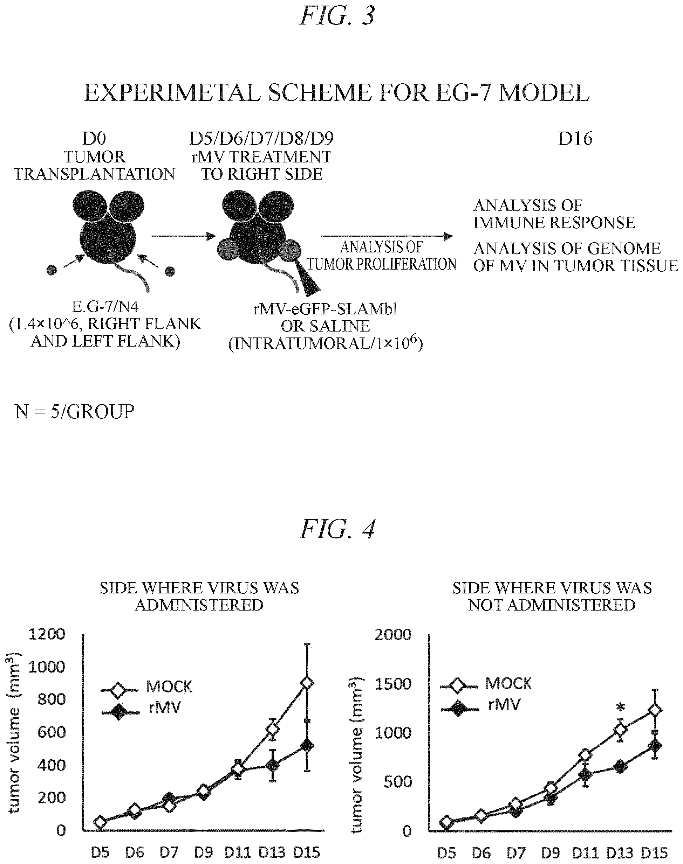

The protocol of the experiment is shown in FIG. 3. Specifically, 1.4 × 10⁶ E.G-7 cells (a T cell lymphoma cell line, ova-expressing tumor, obtained from ATCC) were subcutaneously transplanted into both sides of the flank region of C57BL/6J mice (n = 5) (the day of transplantation was denoted as D0). Thereafter, while raising the mice in a clean environment, 1 × 10⁶ TCID₅₀ of rMV-SLAMblind (rMV-EGFP-SLAMblind) retaining an EGFP gene was administered only to the inside of the right transplantation site on each day at 5 days after transplantation (D5), 6 days after transplantation (D6), 7 days after transplantation (D7), 8 days after transplantation (D8), and 9 days after transplantation (D9), but nothing was administered to the right transplantation site. RMV-EGFP-SLAMblind was prepared according to the method already reported (Patent Document 1). Regarding the size of the tumor, the major and minor axes were measured with a caliper up to D15, the test was terminated on D16, and the transplanted tumor tissue was collected by euthanizing the mouse.

The increase in the tumor volume during the experiment is summarized in FIG. 4. Similarly, as in the case of Example 1, it was shown that the tumor volume on the side where the virus was administered is significantly suppressed by the inherent action of the oncolytic virus (direct anti-tumor effect, left in FIG. 4), and it was shown that the tumor volume on the side where the virus was not administered, the increase in the tumor volume was suppressed even though the oncolytic virus was not administered into the tumor (right in FIG. 4).

In order to elucidate the action mechanism of the anti-tumor effect in the non-virus-administered tumor tissue, an attempt was made to detect the virus genome in the tumor tissue collected on D16. 2 µg of total RNA was subjected to RT-PCR, and after the reaction was terminated, 4 µL of the template diluted 4-fold was subjected to real time PCR using a Thunderbird SYBR qPCR mix and a primer set for the MV genome. After the reaction, the presence or absence of the virus genome was analyzed by an electrophoresis method. The primer set used for cDNA synthesis was an RT-1 primer (ACCAAACAAAGT), and for PCR, an MV-genome-F1 primer (5'-CAATCAATGATCATATTCTAGTACAC-3') and an MV genome-R1 primer (5'-TATAATAATGTGTTTGATTCCTCTG-3') were used.

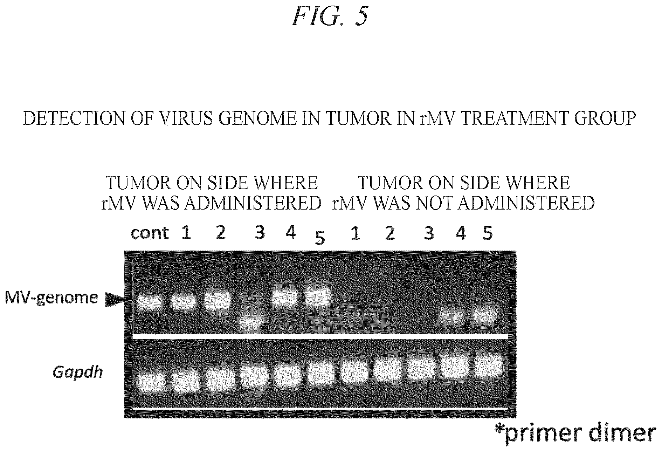

As a result, a sequence derived from the genome of the measles virus was detected from the tumor tissue on the side where the virus had been administered (left in FIG. 5); however, a sequence derived from the genome of the measles virus was not detected from the tumor tissue on the side where the virus was not administered (right in FIG. 5). In FIG. 5, * in lane 3 of the tumor on the side where rMV was administered and lanes 4 and 5 of the tumor on the side where rMV was not administered indicate that the above-described primers form a dimer.

From this result, it was shown that the suppression of tumor volume of the tumor tissue on the side where the virus was not administered, as shown on the right of FIG. 4, occurs by a mechanism different from the direct anti-tumor effect due to the infection of the virus (secondary anti-tumor effect).

### [Example 3] Examination of mechanism of secondary anti-tumor effect of oncolytic gene-modified measles virus (2)

In the present example, the constitution of the tumor-infiltrating immune cells infiltrated into the tumor tissue collected as a result of the experiment of Example 2 was checked in order to examine the action mechanism of the anti-tumor effect of the oncolytic gene-modified measles virus used in the present invention, at the non-virus-administered tumor tissue, where the anti-tumor effect was revealed in Example 1 and Example 2.

In order to elucidate the action mechanism of the anti-tumor effect in the non-virus-administered tumor tissue, various immune cells in the lymph node in the vicinity of the tumor tissue collected on D16 were subjected to detection. Regarding the method, cells were stained with an antibody against a surface antigen of each of various immune cells and then analyzed by the BD FACSVerse flow cytometry.

Infiltrating immune cells were collected from the tumor tissue on the side where the virus had been administered, and NK cells (NK1.1-positive cells), NKT cells (NK1.1-positive and CD3-positive), CD3-positive T cells, CD4-positive T cells, and CD8-positive T cells were subjected to staining of cell surface antigens to analyze each of the numbers of the above cells using a flow cytometry method. CD44 was used as an activation marker of NK cells and T cells, and CD62L was used as a memory T cell marker. In addition, CD44hiCD62L-positive T cells were analyzed as central memory cells, and CD44hiCD62L-negative T cells were analyzed as effector/effector memory cells. Further, the number of activated NK cells and the number of activated T cells was analyzed by staining Granzyme-B and IFN-y, which are cytokines serving as an indicator of activation of NK cells and T cells. On the other hand, the number of immune cells in the tumor tissue on the side where the virus was not administered was also analyzed in the same manner.

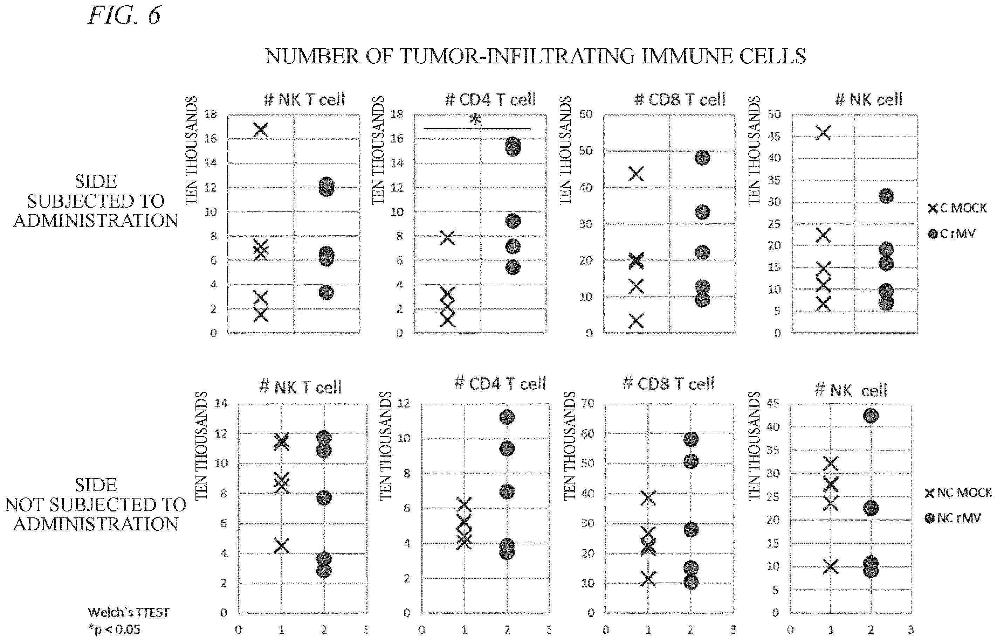

As a result of comparing each of the numbers of the NKT cells, the CD4-positive T cells, the CD8-positive T cells, and the NK cells in the tumor tissue on the side where the virus was administered and the tumor tissue on the side where the virus was not administered, cell infiltration was significantly observed regarding only the CD4-positive T cells in the virus-administered group; however, in the virus-administered group, such a tendency that the tumor tissues on both sides had a large number of infiltrating immune cells was observed (FIG. 6).

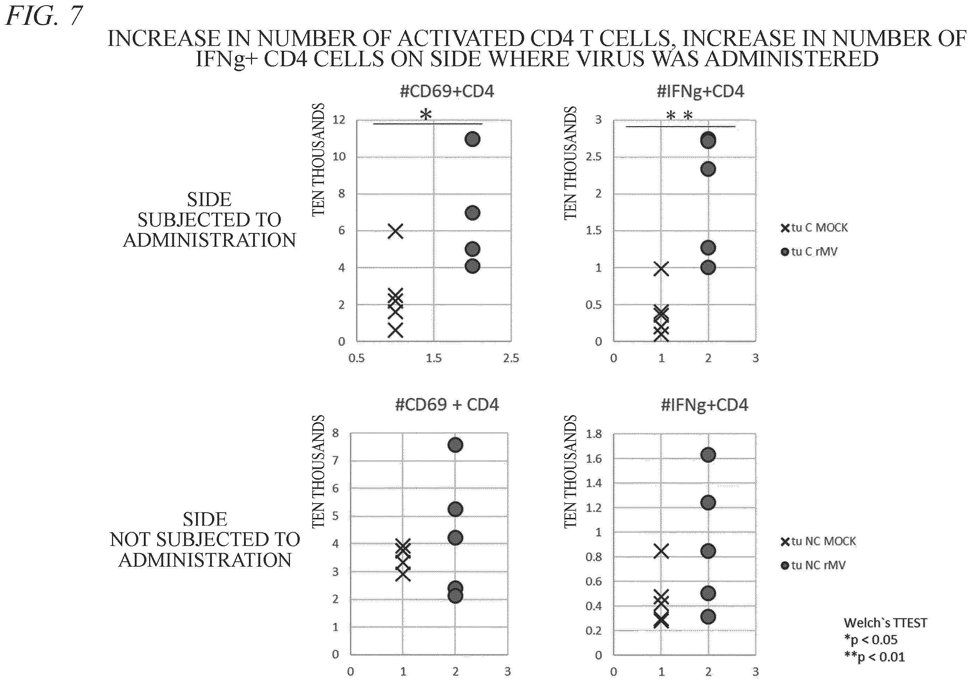

Next, as a result of analyzing the number of activated T cells in the same manner, it was shown that a large number of CD69+ CD4+ T cells and a large number of IFN-y+ CD4 T cells significantly infiltrate the tumor tissue on the side where the virus was administered, in the virus-administered group. A similar tendency was also observed in the tumor tissue on the side not subjected to administration (FIG. 7).

From the state of these various immunocompetent cells, it was suggested that as a result of the cell death of the tumor cells due to the virus administered, CD4-positive T cells, CD8-positive T cells, and NK cells are activated in the tumor tissue on the side where the virus was administered, and these cells also move to the tumor tissue on the side where the virus was not administered, thereby inducing oncolytic virus-independent proliferation suppression or cell death in the tumor tissue on the side where the virus was not administered.

### [Example 4] Examination of mechanism of secondary anti-tumor effect of oncolytic gene-modified measles virus (3)

In the present example, the constitution of the immune cells in the draining lymph node in the vicinity of the tumor tissue collected as a result of the experiment of Example 2 was checked in order to examine an action mechanism of the anti-tumor effect of the oncolytic gene-modified measles virus used in the present invention, at the non-virus-administered tumor tissue, where the anti-tumor effect was revealed in Example 1.

In order to elucidate the action mechanism of the anti-tumor effect in the non-virus-administered tumor tissue, various immune cells in the draining lymph node in the vicinity of the tumor tissue collected on D16 were subjected to detection. The method was the same as described above, and the cells were analyzed by flow cytometry after the immunostaining.

First, the number of cells that highly expressed an activation marker CD44 on NK cells, NK T cells, CD4 T cells, and CD8 T cells, which are present in a lymph node in the vicinity of the tumor (draining LN), was measured.

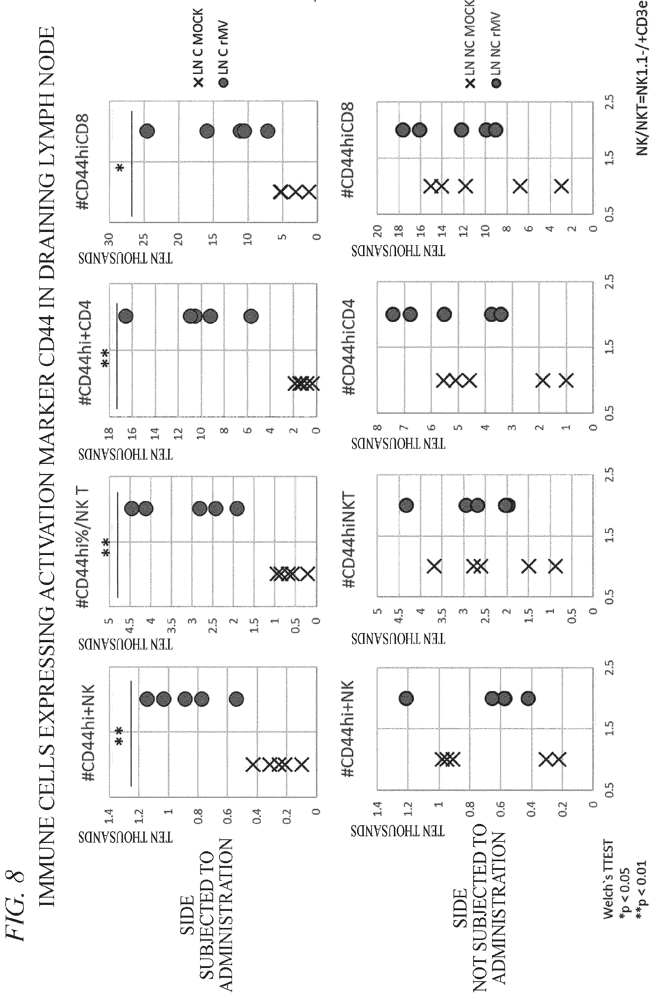

The results are shown in FIG. 8. In the lymph node in the vicinity of the tumor on the side where the virus was administered, a significant increase in the numbers of activated NK cells, NK T cells, CD4 T cells, and CD8 T cells, which highly expressed CD44, was observed. On the other hand, in the draining lymph node on the side where the virus was not administered, no significant difference was observed although there was a tendency of an increase in cell number for each of the cells.

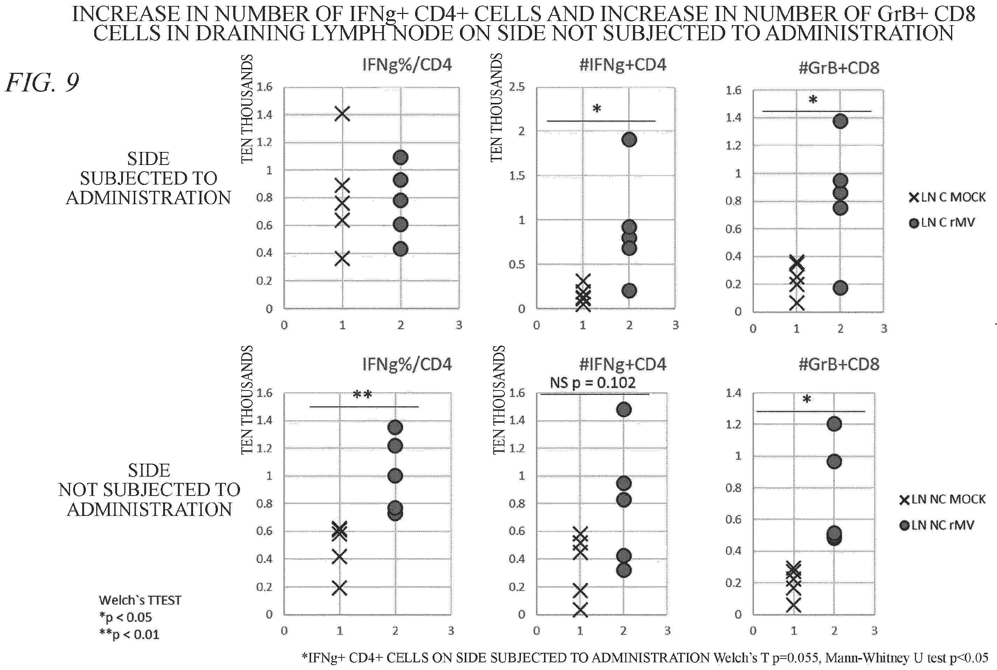

Next, as a result of analyzing the activation of T cells present in the tumor in the vicinity of the draining lymph node using the expression of IFN-γ or GrB as an indicator, a significant increase in the number of IFN-γ-positive CD4 T cells and the number of GrB-positive CD8 T cells in the draining lymph node on the side where the virus was administered was observed (center and right graphs in the upper part of FIG. 9). That is, it was shown that the number of active type CD4 T cells and the number of CD8 T cells has increased.

On the other hand, although the number of IFN-γ-positive CD4 T cells was increased in the draining lymph node on the side where the virus was not administered, no significant increase was observed (p = 0.102) (center in the lower part of FIG. 9).

However, since the proportion of IFN-γ-positive cells in CD4 T cells was significantly increased left in the lower part of FIG. 9), it was revealed that the proportion of active type T cells is increased. In addition, the number of GrB-positive CD8 T cells (active type CD8 T cells) on the side not subjected to administration was significantly increased (right in the lower part of FIG. 9), and thus it was revealed that the active type CD4 cells and the active type CD8 cells are accumulated in the draining lymph node in the vicinity of the tumor on the side where the virus was not administered.

### [Example 5] Response of immune cells in a case where oncolytic gene-modified measles virus was administered (1)

In the present example, the constitution of the immune cells in the spleen tissue collected as a result of the experiment of Example 2 was checked in order to examine an action mechanism of the anti-tumor effect of the oncolytic gene-modified measles virus used in the present invention, at the non-virus-administered tumor tissue, where the anti-tumor effect was revealed in Example 1.

In order to check the systemic response of immune cells, the various immune cells in the spleen tissue collected on D16 were subjected to detection. In this experiment, the NK1.1+ CD3+ cells were defined as NKT cells, the NK1.1+ CD3- cells were defined as NK cells, the NK1.1- CD4+ cells were defined as CD4+ T cells, and the NK1.1- CD8+ cells were defined as CD8+ T cells, and the cells were stained to calculate the number of the cells.

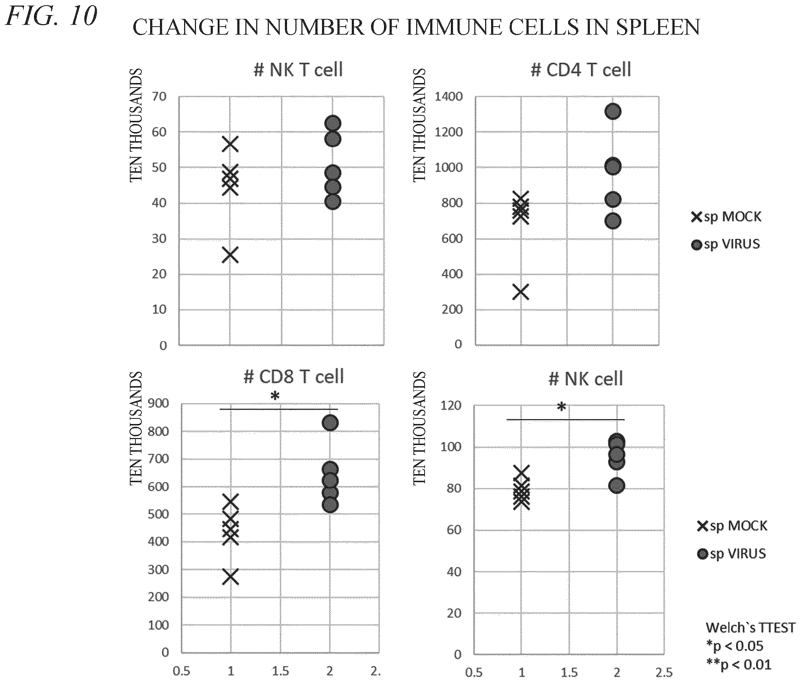

As shown in FIG. 10, a significant increase in the number of cells was observed in CD8 T cells and NK cells. That is, it was revealed that the active type CD8+ T cell and the active type NK cell proliferate and circulate systemically.

Further, the expression system analysis was carried out by comparing the number of effector memory (EM) CD4-positive (CD4+) T cells, the number of central memory (CM) CD4-positive T cells, the number of GrB-positive and CD8-positive (CD8+) T cells, and the number of IFN-γ-positive and CD8-positive T cells in the spleen tissue.

In general, the central memory T cell is a cell that expresses a chemokine receptor CCR7 or an adhesion factor CD62L and is present mainly in a T cell region of secondary lymphoid tissues such as a lymph node and the spleen, and the central memory T cell has a function of producing IL-2 thereby rapidly proliferating in a case of being exposed again to an antigen to which it has been exposed in the past. On the other hand, in general, the effector memory T cell has a decreased expression of adhesion factors such as CCR7 and CD62L, is present mainly in a local inflammatory site (for example, lung, liver, or intestinal tract), and has a function of producing a large amount of cytokines such as IL-4, IFN-γ, and IL-5 in response to stimulation with an antigen to which the effector memory T cell has been exposed in the past. In a case where these two kinds of memory T cells survive for a long period of time, it is possible to rapidly trigger an immune response even in a case where the same antigen (for example, a pathogen) reappears.

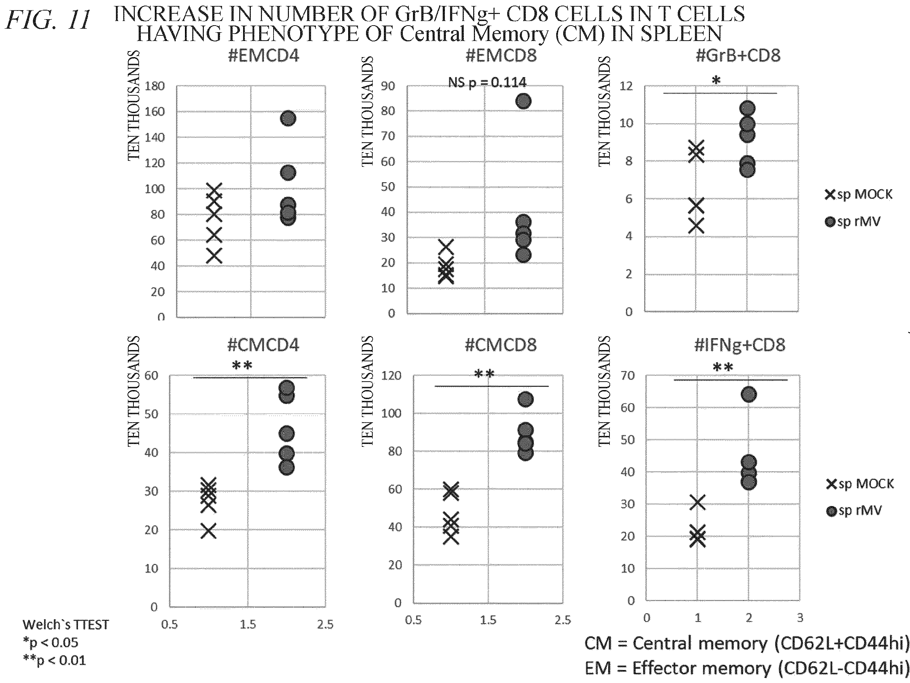

As a result, the comparison with the case of the spleen tissue of the group administered with MOCK (negative control) revealed the following facts. · As a result of the analysis of GrB and IFN-y, the numbers of GrB+ CD8 T cells and IFN-γ+ CD8 T cells (both are active type CD8 T cells), which are T cells expressing GrB and IFN-y which are indicators of activation, were significantly increased (right graphs in the upper and lower parts of FIG. 11).
· In addition, as a result of analyzing central memory cells (CM; CD62L+ CD44hi T cells) that are required to exhibit strong immune power against cancer cells, it was revealed that both CMCD4 T cells and CMCD8 T cells are significantly increased (left and center in the lower part of FIG. 11).
· On the other hand, the number of CD4 T cells did not increase although an increase in the number of CD8 T cells having an expression system of effector/effector memory cells was observed.

### [Example 6] Response of immune cells in a case where oncolytic gene-modified measles virus was administered (2)

rMV-SLAMblind treatment was shown to rapidly and significantly induce the IFN-γ-producing type 1 CD4+ T cells (Th1). The Th1 cell is a cell that plays an extremely important role in establishing an anti-tumor response by assisting the CTL function and the differentiation of memory CD8+ T cells. Therefore, in the present example, it was analyzed whether or not the tumor antigen-specific CD8+ T cells were induced by rMV-SLAMblind treatment.

E.G-7 cells were transplanted into B6 mice, and subsequently, a MOCK or rMV-SLAMblind treatment was carried out on the 3rd day and 5th day after the tumor transplantation (n = 5/group). On the 13th day, the mice were euthanized, and the TDLN of the animals was collected.

To evaluate the tumor antigen-specific T cells, immature bone marrow derived dendritic cells (BMDCs) were prepared from bone marrow cells of C57BL/6J mice. In brief, bone marrow cells derived from the femur of an adult mouse were cultured in cRPMI to which 20 ng/mL of GM-CSF (PeproTech) was added. After 3 days, fresh cRPMI and GM-CSF were supplemented to the culture medium, and the cells were further cultured for 3 days.

On the 6th day, immature BMDCs were collected and stimulated as follows. In order to stimulate immature DC having a tumor antigen, a tumor cell lysate to which heat shock was applied was prepared as follows. In brief, E.G-7 cells were incubated at 42°C for 1 hour and then incubated at 37°C for 2 hours. After the incubation, the cells were washed with PBS, the cells were resuspended in PBS at a density of 1.5 × 10⁷/mL, the cell suspension was lysed by carrying out three cycles of freeze-thawing and centrifugal separation at 20,400 g for 10 min, and the supernatant was stored at -30°C until use.

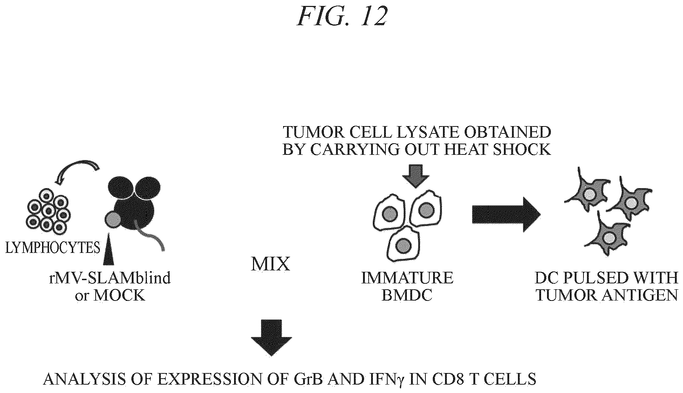

The immature BMDCs were stimulated by being treated, overnight in the presence of 20 ng/mL of GM-CSF, with a tumor cell lysate (tumor antigen-pulsed DC) obtained by carrying out heat shock, at a ratio of 4:1 (tumor cell:DC). The stimulated immature BMDCs were mixed with lymphocytes obtained from the E.G-7-bearing mouse subjected to a treatment with rMV-SLAMblind or the E.G-7-bearing mouse subjected to a MOCK treatment. After co-culturing overnight, the expression of IFN-y and GrB in the CD8+ T cells of these animals was analyzed by flow cytometry (FIG. 12).

The maturation state of the DC was monitored by flow cytometry (data not shown) based on the expression of CD11c (CD11c-FITC (clone; HL3) (BD Biosciences)), I-A/IE (I-A/I-E Pacific Blue (clone; M5/114.15.2) (BioLegend)), CD80 (CD80-PerCP-Cy5.5 (clone; 16-10A1) (BioLegend)), and CD86 (CD86-APC (clone; GL1) (BD Biosciences)).

After the stimulation, the DC was treated with 50 µg/mL of mitomycin C (WAKO) for 1 hour and then washed twice with cRPMI. Further, lymphocytes were prepared from a tumor-draining lymph node (TDLN) of a mouse having an E.G-7 tumor, which had been subjected to a MOCK treatment, or a mouse having an E.G-7 tumor, which had been subjected to an rMV-SLAMblind-treatment and then labeled with efluor450 (eBioscience) to identify the cells in the DC mixture. Next, the lymphocytes and the DCs were mixed at a ratio of 5:1 (lymphocyte:DC). After co-culturing overnight, the cells were stimulated with PMA and ionomycin for 4 hours in the presence of brefeldin A and subsequently stained and subjected to flow cytometry.

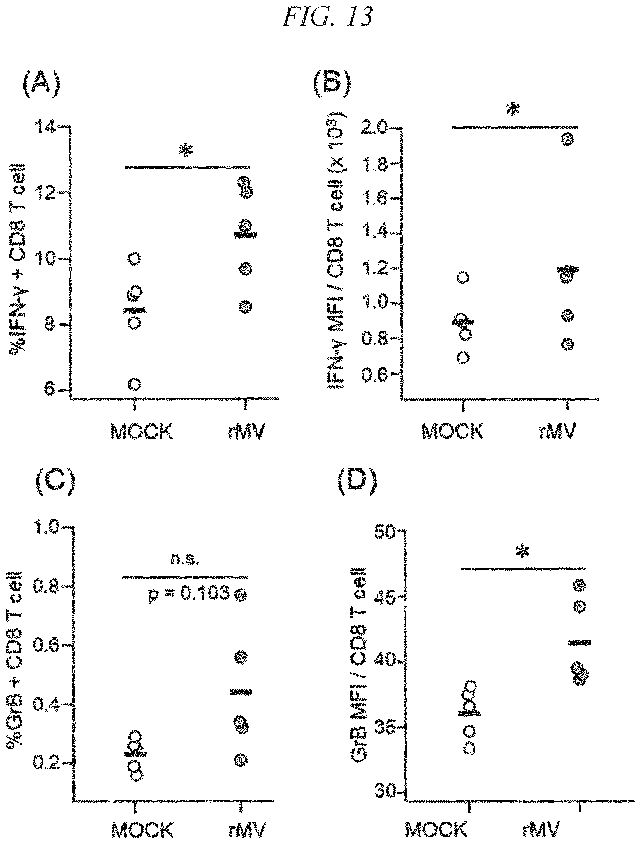

Compared with the proportion of CD8+ T cells obtained from the MOCK-treated mouse, a higher proportion of CD8+ T cells obtained from the mouse treated with rMV-SLAMblind expressed IFN-y and GrB in response to the tumor antigen-pulsed DC.(%IFN-γ in CD8+, p < 0.05 in FIG. 13A and %GrB in CD8+, p = 0.103 in FIG. 13C). In addition, the expression of IFN-γ and GrB was significantly high in the CD8+ T cells collected from rMV-SLAMblind-treated mouse compared with those collected from the MOCK-treated mouse (IFN-γ, p < 0.05; GrB, p < 0.05, in FIG. 13B and 13D).

From these results, it was shown that rMV-SLAMblind treatment induces the tumor antigen-specific CD8+ T cell response.

### [Example 7] Functional analysis of oncolytic virus using gastric cancer cells

### <Material and method>

### · Cell

OCUM-1, NUGC-4, and KatoIII cells were purchased from the Japanese Cancer Research Resources Bank (Tokyo, Japan) and proliferated in Dulbecco's modified Eagle medium (DMEM; Sigma, St. Louis, MO) containing 0.5 mM sodium pyruvate (Life Technologies, Carlsbad, CA), 10% fetal bovine serum (FBS; SAFC Biosciences, Lenexa, KS), streptomycin (100 mg/mL), and penicillin (100 U/mL). GCIY cells were obtained from RIKEN Bioresource Center (Ibaraki, Japan) and cultured in a minimum essential medium (Sigma) containing 15% FBS and an antibiotic. HSC-60, HSC-43, HSC-44PE, HSC-64, MKN28, TMK-1, HSC-39, NKPS, 58As1, 58As9, and HSC-59 were previously established from gastric cancer tissues (refs) and cultured in RPMI 1640 medium (Sigma) containing 10% FBS and an antibiotic. Vero cells (Vero/N4 cells) expressing human Nectin-4 were proliferated in DMEM containing 10% FBS and an antibiotic. All the cells were cultured at 37°C and 5% CO₂.

### · Preparation of virus

rMV-EGFP-SLAMblind was proliferated in the Vero/N4 cells. Each virus titer was determined as TCID₅₀ using the Vero/N4 cells by the Reed-Munch method.

### · Flow cytometry

Cells were washed with PBS and detached with 0.025% trypsin/0.24 mM ethylenediaminetetraacetic acid (EDTA). After centrifugal separation at 3,300 × g for 1 minute, the cells were resuspended in 100 µl of PBS containing 2% FBS and incubated on ice for 30 minutes together with 0.5 µg of an anti-human Nectin-4 monoclonal antibody (R&D Systems, Minneapolis, MN) and an anti-human SLAM antibody (BioLegend, San Diego, CA).

· Next, the cells were once washed and then stained with 0.1 µg of an Alexa 488-conjugated anti-mouse IgG (Molecular Probes, Eugene, OR) on ice for 30 minutes. Finally, the cells were washed twice with PBS containing 2% FBS and then resuspended in PBS containing 7-aminoactinomycin D (Beckman Coulter Immunotech, Massielle, France). The fluorescence intensity was measured with FACSCalibur (BD Biosciences, San Jose, CA, USA), and the data were analyzed with FlowJo software ver. 9.7.5 (TreeStar, San Carlos, CA).

### · Viral infection of gastric cancer cell line

Cells were cultured in a 12-well plate and infected with rMV-EGFP-SLAMblind at an MOI of 0.1. The infection of the virus was observed with an all-in-one fluorescence microscope (BZ-710; Keyence, Osaka, Japan) on the 5th day post infection (5 dpi).

### · Cell proliferation assay

Cells were infected with rMV-EGFP-SLAMblind at an MOI of 1. Thereafter, the cells were seeded at a density of 5 × 10³ cells per well in 200 µL of a culture medium in a 96-well plate and cultured at 37°C.

The cell survival rate was measured at 1, 3, 5, and 7 dpi using a WST-1 cell proliferation kit (Takara BIO INC., Otsu, Shiga, Japan) according to the manufacturer's protocol. The survival rate of the virus-infected cells was calculated as a value that was obtained by dividing the average absorbance value by the average absorbance value of the non-infected cells and was indicated in terms of percentage.

### · Xenotransplantation model

The animal experiments were approved by the Animal Experiment Committee of the University of Tokyo. HSC-60 cells (5 × 10⁶) or HSC-43 cells (3 × 10⁶) were suspended in 50 µL of Hank's balanced salt solution (HBSS; Thermo Fisher Scientific, Wilmington, DE) containing 2% FBS, and the suspension was mixed with 50 µl of Matrigel (BD Biosciences). The cell suspension was injected into the lateral abdomen of a 7-week-old male NSG (NOD-scidIL2rγnull) mouse for the HSC-60, and into the lateral abdomen of a 6-week-old male SCID mouse (CB-17/Icrscid/scidJc1) for the HSC-43 (each mouse was purchased from Clea Japan (Tokyo, Japan)).

10⁶ TCID₅₀ of rMV-EGFP-SLAMblind (n = 5) or HBSS (n = 5) was administered intratumorally to the mice three times at one-week intervals. The diameter of the tumor was measured with a caliper every 3 to 4 days for 28 days after the first administration. The tumor volume was calculated based on the expression (width × width × length)/2. All mice were euthanized 28 days after the first virus administration, and tumor samples were collected. The fluorescence of EGFP in the isolated tumor was observed with a fluorescence microscope (MVX10; Olympus, Tokyo, Japan).

### · Retroviral transduction

A plasmid expressing a firefly luciferase was generated as follows. The coding region of the firefly luciferase was amplified with Phusion High-Fidelity DNA Polymerase (New England Biolabs, Ipswich, MA) using primers specific to the firefly luciferase (5'-aaa ccg gtg cca cca tgg aag acg cca aaa aca t -3' and 5'-ttg gat cct tac acg gcg atc ttt ccg c -3').

The PCR product was cloned between the AgeI site and the BamHI site of the pQCXIH vector (Clontech Laboratories, Palo Alto, CA), which was named pQCXIH-luc. PlatA cells were transfected with the pQCXIH-luc using polyethyleneimine "Max" (Mw 40,000)-high titer linear PEI (Polysciences, Inc., Warrington, PA) according to the manufacturer's instructions.

Two days later, the supernatant was collected and filtered through a 0.45 µm Acrodisc syringe filter (Pall Corporation, Port Washington, NY). The virus was incubated with HSC-60 cells in the presence of 8 µg/mL of polybrene (Sigma). The transduced cells were cultured for 2 weeks in a culture medium containing 200 to 400 µg/mL of hygromycin B (Thermo Fisher Scientific).

### · In vivo visualization analysis of anti-tumor activity in peritoneal metastasis model

HSC60-Luc cells (3 × 10⁶) were suspended in 300 µL of HBSS containing 2% FBS and intraperitoneally inoculated into a 7-week-old male NSG mouse. Five days after the inoculation, 2 × 10⁶ TCID₅₀ of rMVEGFP-SLAMblind was intraperitoneally administered to the mouse. The bioluminescence analysis was carried out at the indicated dpi.

2 mg of D-luciferin (Gold Biotechnology, St. Louis, MO, USA) diluted with 200 µL of PBS was infected into the abdominal cavity of the mouse. An image was acquired using a Xenogen IVIS 100 system (Xenogen, Alameda, CA, USA). Using Living Image Software ver. 2.5 (Xenogen), the luciferase activity was quantified in terms of total photons/s/cm²/sr.

### · Statistical analysis

The statistical analysis of the in vivo experiment was carried out using the Welch's t-test by MacTokei-Kaiseki ver. 1.5 software (Esumi, Tokyo, Japan).went. P < 0.05 was considered to be statistically significant.

### <Expression of Nectin-4 in scirrhous gastric cancer cell line and non-scirrhous gastric cancer cell line>

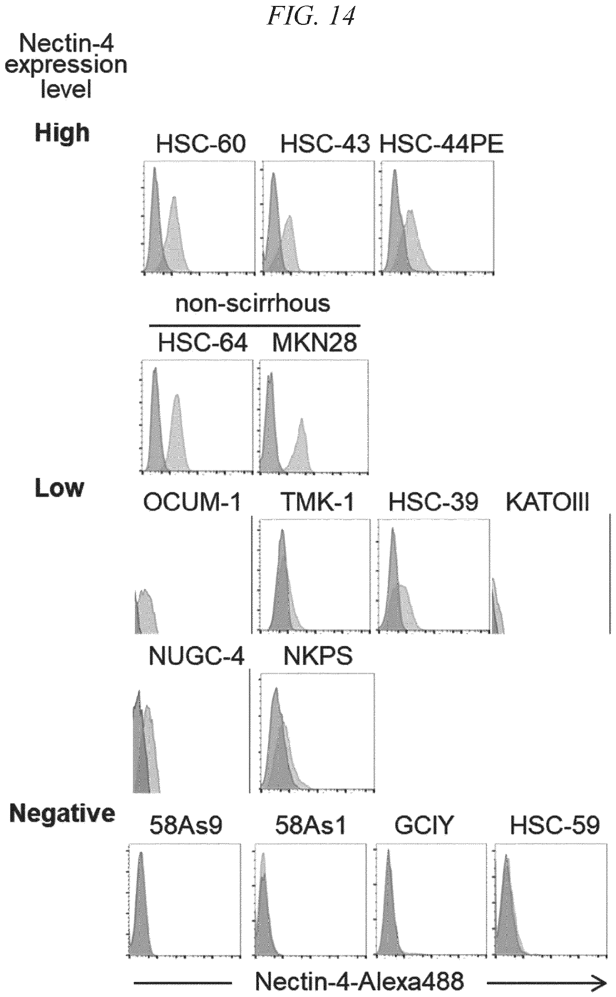

In order to investigate whether Nectin-4, which is a receptor of rMV-SLAMblind, is expressed on the cell surface of the gastric cancer cell, 13 kinds of SGC cell lines and 2 kinds of non-SGC cell lines were stained with an anti-human Nectin-4 monoclonal antibody (light gray histogram) or a control IgG (dark gray histogram) and then incubated with an Alexa-Fluor-488-conjugated anti-mouse antibody to analyze the expression of Nectin-4 by flow cytometry. The cells were classified into three groups of a high expression group (denoted as "High" in FIG. 14 to FIG. 16), a low expression group (denoted as "Low" in FIG. 14 to FIG. 16), and a non-expression group (denoted as "Negative" in FIG. 14 to FIG. 16) according to the expression level of Nectin-4 (FIG. 14). Data was analyzed using FlowJo software.

Nectin-4 was detected in 9 kinds of SGC cell lines (69%) and 2 kinds of non-SGC cell lines (100%); however, it was not detected in 4 kinds of SGC cell lines. Among the Nectin-4-expressing cell lines, high expression was observed in 3 kinds of SGC cell lines (HSC-60, HSC-43, and HSC-44PE) and 2 kinds of non-SGC cell lines (HSC-64 and MKN28). The expression level of Nectin-4 in the other 6 kinds of SGC cell lines (OCUM-1, TMK-1, HSC-39, KATOIII, NUGC-4, and NKPS) was low.

From these results, it is suggested that although Nectin-4 is highly expressed in SGC cells, the expression levels are different between the cell lines.

### <Infectivity of rMV-SLAMblind in scirrhous gastric cancer cell line and non-scirrhous gastric cancer cell line>

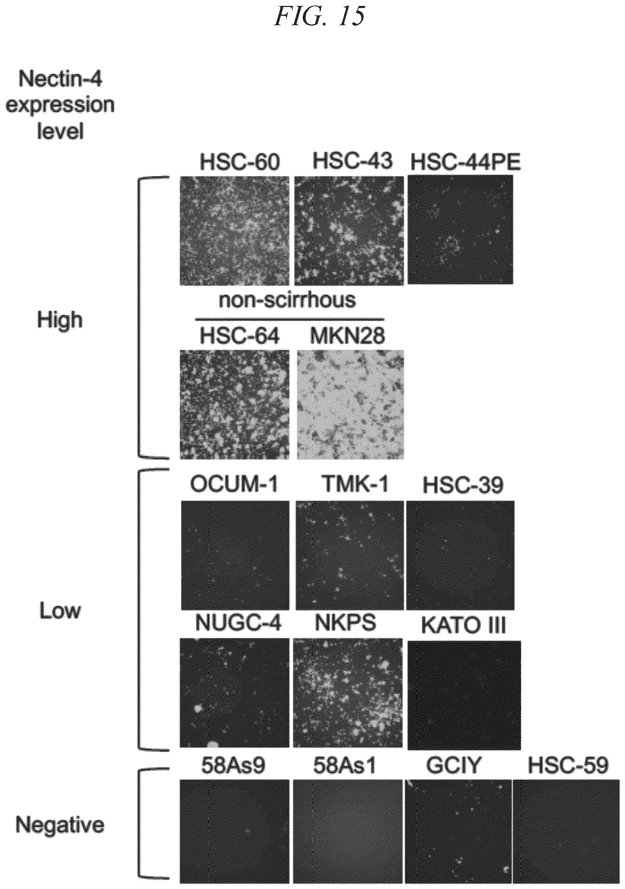

In order to investigate the sensitivity to rMV-SLAMblind, the scirrhous gastric cancer cell line and the non-scirrhous gastric cancer cell line were infected with rMV-SLAMblind (rMV-EGFP-SLAMblind) expressing EGFP at an MOI of 0.1 (FIG. 15). The fluorescence of EGFP was observed at 5 dpi under a fluorescence microscope, and representative data are shown. Magnification: × 100.

The virus-derived EGFP signal was detected in most of the Nectin-4 positive cell lines, and it was hardly detected in the Nectin-4 negative cell lines. In particular, four kinds (HSC-60, HSC-43, HSC-64, and MKN28) of the five kinds of cell lines having a high expression level of Nectin-4 showed a strong signal (FIG. 15, "High").

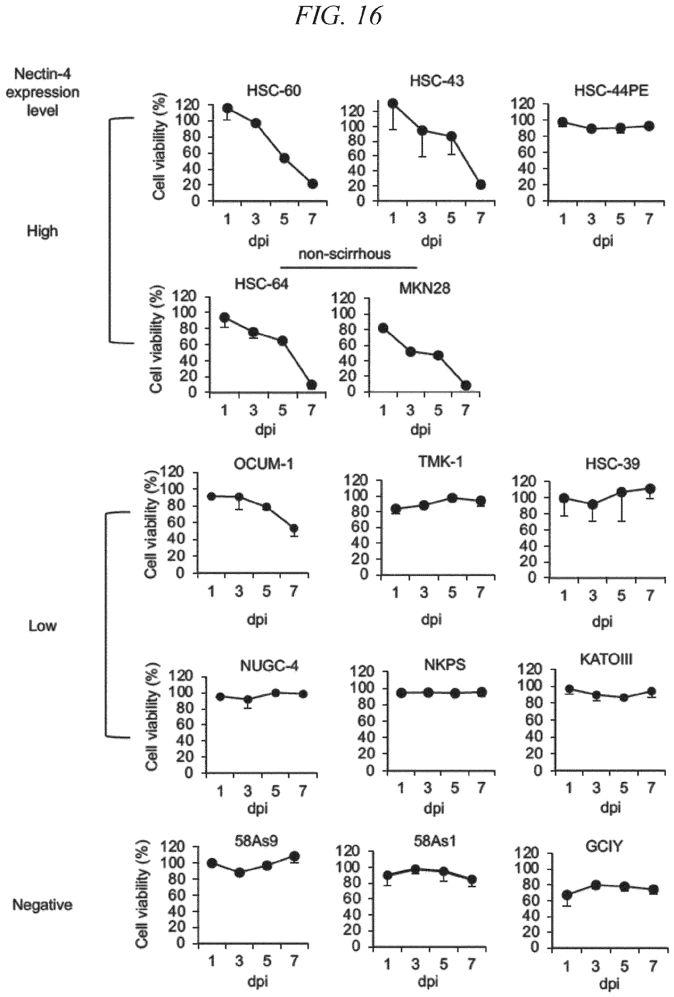

Next, in order to measure the cytotoxicity, cells were infected with rMV-SLAMblind at an MOI of 1, and then a cell survival rate assay was carried out at 1, 3, 5, and 7 dpi using a water-soluble tetrazolium salt (WST) assay system (FIG. 16). The error bar indicates the SD of three independent experiments.

rMV-SLAMblind killed 75% or more of HSC-60 cells, HSC-43 cells, HSC-64 cells, and MKN28 cells and approximately 50% of OCUM-1 cells by the 7th day post infection (7 dpi). In HSC-44PE, despite the high expression level of Nectin-4, the decrease in the cell survival rate was not observed. The survival rate of the cell lines in which Nectin-4 was expressed at a low level or at a level of 0 was not decreased except for OCUM-1.

From these results, it was suggested that rMV-SLAMblind exhibits cytotoxicity against the scirrhous gastric cancer cells and the non-scirrhous gastric cancer cells in vitro, where the cytotoxicity is substantially correlated with the expression level of Nectin-4.

### <Anti-tumor activity of rMV-SLAMblind in subcutaneous xenotransplantation model>

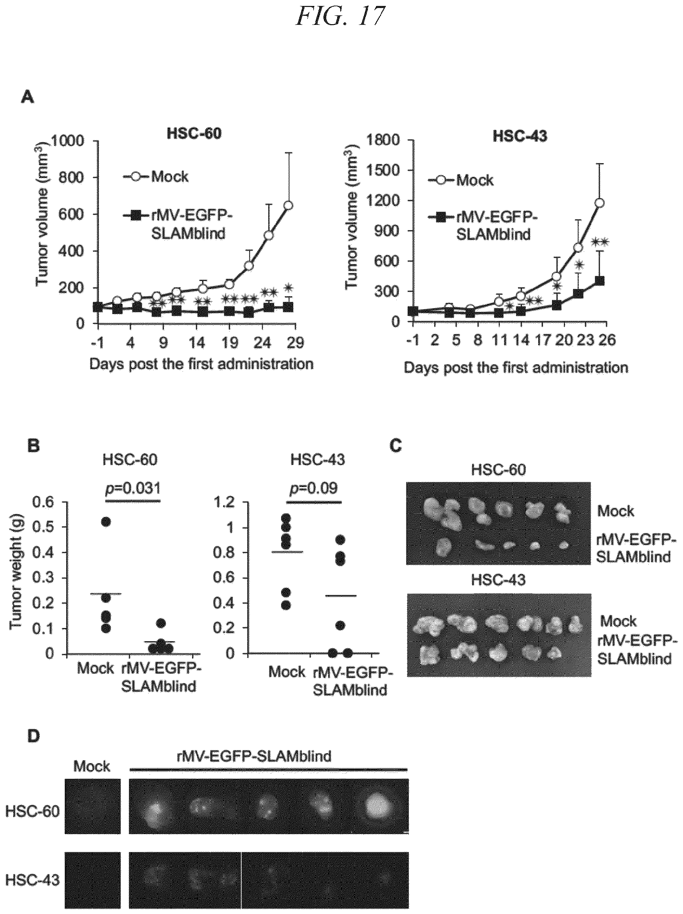

In order to investigate the anti-tumor activity of rMV-SLAMblind in vivo, each of human xenotransplantation models of HSC60 cells and HSC43 cells were produced by transplanting the HSC60 cells and the HSC43 cells into the subcutaneous region of a NOD-scid IL2rγnull (NSG) mouse and a severe combined immunodeficiency (SCID) mouse. After the tumor was established and then reached 90 mm³ (on the 9th day for HSC-60 and the 5th day for HSC-43), rMV-EGFP-SLAMblind at a dose of 10⁶ TCID₅₀ or HBSS as a control was administered intratumorally three times at one-week intervals, that is, 7 days and 14 days after the first inoculation. Two groups were compared using the Welch's t-test. The error bar indicates SD. *P < 0.05 was considered to be statistically significant. *P < 0.05, **P < 0.01. The administration of rMV-EGFP-SLAMblind showed a significant anti-tumor activity in both the transplantation models of HSC-60 cells and HSC-43 cells (FIG. 17A).

28 days after the first administration, each mouse was euthanized, and an autopsy was carried out to collect a tumor sample. The weight of the collected tumor was measured on the 28th day. The error bar indicates SD. The P value was indicated according to the Welch's t-test. The tumor size of the HSC-60 xenotransplantation model of the virus treatment group was obviously smaller, and the tumor weight was also smaller than that of the control group (FIG. 17B and FIG. 17C). In FIG. 17C, the upper sample was a tumor of the control group, and the lower sample was a tumor of the virus inoculation group.

The same tendency was also shown in the HSC-43 model. That is, the isolated tumor of the mouse treated with HBSS (left panel) or rMV-SLAMblind (right panel) at 28 dpi was observed with an immunofluorescence microscope. Further, the fluorescence of EGFP, which indicates the proliferation of the virus, was detected only in the tumor administered with rMV-EGFP-SLAMblind (FIG. 17D).

From these results, it was suggested that rMV-SLAMblind has an anti-tumor activity in vivo.

### <Oncolytic activity against peritoneal metastasis model>

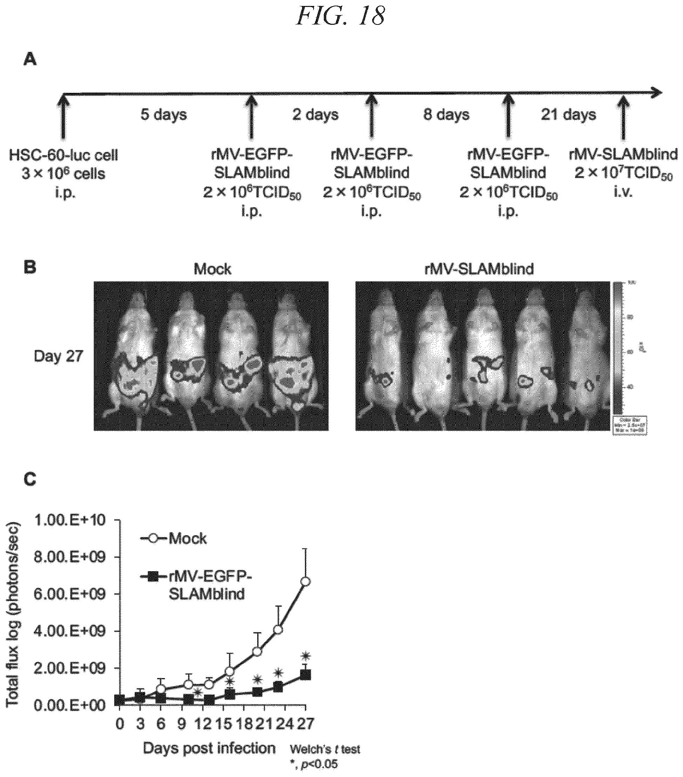

The SGC was characterized by progressive infiltration and a high frequency of peritoneal metastasis. In order to investigate whether rMV-SLAMblind could exhibit oncolytic activity against peritoneal dissemination, 3 × 10⁶ cells of HSC-60 cells (HSC60-Luc) expressing luciferase were intraperitoneally injected into NSG mice to establish a peritoneal dissemination xenotransplantation model. Five days later, after the luciferase activity in the abdominal cavity was increased, 2 × 10⁶ TCID₅₀ of rMV-SLAMblind or HBSS was administered intraperitoneally three times, 0, 2, and 8 days after the first administration (FIG. 18A).

The proliferation of the tumor was monitored by a Xenogen IVIS100 system until the 27th day. As shown in FIG. 18B, although the tumor cells of the control group were widely diffused in the abdominal cavity, the tumor cells of the virus treatment group were significantly limited. Representative data of luciferase activity 27 days after the first virus inoculation are shown.

Further, the value of the luciferase activity at each time point is shown. Two groups were compared using the Welch's t-test. The error bar indicates SD. *P < 0.05 was considered to be statistically significant. The value of the luciferase activity was significantly lower in the virus treatment group than in the control group (FIG. 18C).

All of the mice in the virus-treatment group survived until 43 days post the first administration, whereas half of the mice died 34 days after the first administration, and those with tumor overgrowth were euthanized 41 days after the first administration. The survived mice were euthanized and analyzed 43 days after the first administration. As a result, the control mouse developed hemorrhagic ascites, while the virus-treated mouse did not (data not shown).

From these results, it was suggested that the intraperitoneal administration of rMV-SLAMblind might exhibit an anti-tumor activity against peritoneal dissemination.

### [Example 8] Examination of mechanism of secondary anti-tumor effect of oncolytic gene-modified measles virus (4)

In the present example, in order to examine an action mechanism of the anti-tumor effect of the oncolytic gene-modified measles virus used in the present invention, at the non-virus-administered tumor tissue, where the anti-tumor effect was revealed in Example 1 and Example 2, the effect of various immune responsive cells induced to be activated by the administration of the oncolytic gene-modified measles virus, on the anticancer therapy, was investigated.

The administration of the oncolytic gene-modified measles virus induces the activation of NK cells, CD4+ T cells, and tumor antigen-specific CD8+ T cells. In order to investigate whether or not the induced immune response of these cells affects the cancer therapeutic effect, an in vivo cell-specific depletion study was carried out.

Specifically, first, B16F10/N4 cells were transplanted into B6 mice (the transplantation date is denoted as D0). Thereafter, while raising the mice in a clean environment, the specific immune cell population was depleted by injecting a rat IgG2B (isotype control), an anti-CD8 antibody, an anti-CD4 antibody, and an anti-NK1.1 antibody on each at 3 days after the transplantation (D3), 6 days after the transplantation (D6), 10 days after the transplantation (D10), and 14 days after the transplantation (D14) (n = 8 per antibody-administered group). In addition, rMV-SLAMblind was administered on each at 4 days after the transplantation (D4), 5 days after the transplantation (D5), 6 days after the transplantation (D6), 8 days after the transplantation (D8), and 10 days after the transplantation (D10). In addition, the tumor volume was measured over time for each mouse starting 5 days after the transplantation, and the growth of the tumor was analyzed. The depletion of the subset of immune cells was checked 2 days after the second antibody injection.

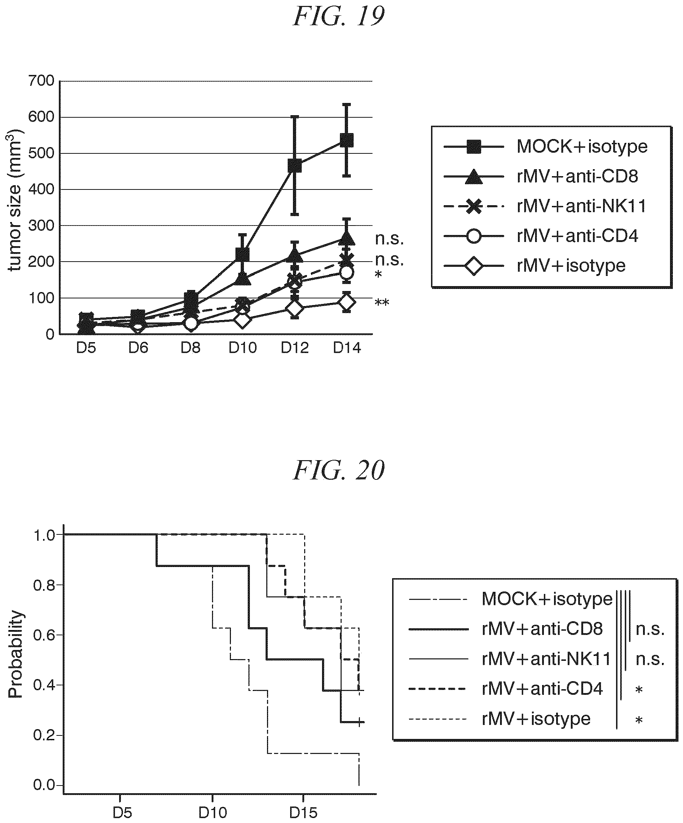

FIG. 19 shows the results obtained by measuring the tumor volume (average value ± SEM) of each group over time, and FIG. 20 shows the results obtained by measuring the survival rate of each group over time. Statistical significance was analyzed according to the Kruskal-Wallis test, and the P value was calculated according to the Steel test (in the figure, * = p < 0.05, ** = p < 0.01). As shown in FIG. 19, the tumor volume 14 days after the transplantation (D14) was significantly suppressed in the rMV-SLAMblind + isotype control-treated mouse (rMV + isotype) group compared with the MOCK + isotype control-treated mouse (MOCK + isotype) group (D6 to D14, P < 0.01), and it was also significantly suppressed in the rMV-SLAMblind + anti-CD4 antibody-treated mouse (rMV + anti-CD4) group although the tumor volume was slightly large compared with the rMV + isotype group. On the other hand, the tumor volumes 14 days after the transplantation (D14) in the rMV-SLAMblind + anti-CD8 antibody-treated mouse (rMV + anti-CD8) group and the rMV-SLAMblind + anti-NK1.1 antibody-treated mouse (rMV + anti-NK1.1) group were smaller than those of the MOCK + isotype group; however, there was no statistically significant difference. In addition, as shown in FIG. 20, the survival rate in the rMV + anti-CD4 group was the same as that in the rMV+isotype group, whereas the survival rate in the rMV + anti-CD8 group and the rMV + anti-NK1.1 group was lower than that in the rMV + isotype group. Specifically, the median value during the survival period (MST) was shortened to 19.5 days in the rMV + anti-CD8 group and was shortened to 22 days in the rMV + anti-NK1.1 group. From these results, it was shown that the depletion of CD8+ T cells and the depletion of NK cells significantly reduce the tumor suppressive function of the rMV-SLAMblind administration, in other words, CD8+ T cells and NK cells are required for the tumor suppressive effect and the cancer therapeutic effect of the rMV-SLAMblind administration. In particular, it was found that among the immune cells activated by the administration of rMV-SLAMblind, activated killer T cells (CD8+ T cells) play the most important role in the secondary anti-tumor effect of rMV-SLAMblind.

### Industrial Applicability

The oncolytic gene-modified measles viruses (rMV-SLAMblind and rMV-V(-)-SLAMblind), which are used in the present invention, are capable of not only directly inducing remarkable cell death in the tumor cells by intratumoral administration (direct anti-tumor effect) but also inducing a strong cell-mediated immunity against tumor cells that have not been directly infected with the virus (metastasized tumor cells, deep-seated tumor cells that spread to a site where the virus cannot be directly infected, and the like) (secondary anti-tumor effect).

### Sequence Listing Free Text

SEQ ID NO: 8: RT-1 primer for cDNA synthesis: ACCAAACAAAGT
SEQ ID NO: 9: MV-genome-F1 primer for PCR:
   CAATCAATGATCATATTCTAGTACAC
SEQ ID NO: 10: MV genome-R1 primer for PCR:
   TATAATAATGTGTTTGATTCCTCTG
SEQ ID NO: 11: Primer specific to firefly luciferase: aaa ccg gtg cca cca tgg aag acg cca aaa aca t
SEQ ID NO: 12: Primer specific to firefly luciferase: ttg gat cct tac acg gcg atc ttt ccg c

## Claims

1. A pharmaceutical composition for inducing a cell-mediated immunity against a tumor cell to subject the tumor cell to a medical treatment, the pharmaceutical composition comprising:
an oncolytic gene-modified measles virus.

2. The pharmaceutical composition according to Claim 1, wherein the tumor cell as a medical treatment target is a tumor cell expressing PVRL4/Nectin4.

3. The pharmaceutical composition according to Claim 1 or 2, wherein a tumor as a medical treatment target is breast cancer, lung cancer, colon cancer, or pancreatic cancer.

4. The pharmaceutical composition according to Claim 1 or 2, wherein the tumor cell as a medical treatment target is a tumor cell that remains even after direct introduction of the oncolytic gene-modified measles virus.

5. The pharmaceutical composition according to Claim 4, wherein the remaining tumor cell is selected from the group consisting of a deep-seated tumor cell, a recurrent tumor cell, and a metastasized tumor cell.

6. The pharmaceutical composition according to Claim 1 or 2, wherein the oncolytic gene-modified measles virus is rMV-SLAM-blind or rMV-V(-)-SLAM-blind.

7. The pharmaceutical composition according to Claim 1 or 2, wherein the oncolytic gene-modified measles virus is used by intravenous administration, direct administration to a tumor mass, or intraperitoneal administration.

8. The pharmaceutical composition according to Claim 1 or 2, wherein the oncolytic gene-modified measles virus also induces the cell-mediated immunity in addition to an oncolytic action.

9. A method of inducing a cell-mediated immunity against a tumor cell remaining in a living body that could not be eliminated even by direct introduction of an oncolytic gene-modified measles virus, or against a tumor cell that has metastasized or recurred, the method comprising:
directly introducing an oncolytic gene-modified measles virus into a tumor cell as a medical treatment target to cause cell death.

10. The method according to Claim 9, wherein the remaining tumor cell is selected from the group consisting of a deep-seated tumor cell, a recurrent tumor cell, and a metastasized tumor cell.

11. The method according to Claim 9 or 10, wherein the oncolytic gene-modified measles virus is rMV-SLAM-blind or rMV-V(-)-SLAM-blind.
